# EUROPEAN PATENT APPLICATION

(11) **EP 4 806 358 A1**
(43) Date of publication of application: **16.09.2026**
(21) Application number: 26163459.6
(22) Date of filing: 10.03.2026
(51) Int. Cl.: A61B 6/00

(54) **TRACKING TARGET POSITION DETECTION DEVICE, RADIOTHERAPY SYSTEM, AND TRACKING TARGET POSITION DETECTION METHOD**

(30) Priority: 11.03.2025 JP 2025038167
(71) Applicant: Hitachi High-Tech Corporation, Minato-ku Tokyo 105-6409 (JP)
(72) Inventor: HIRAYAMA, Shusuke, Tokyo, 100-8280 (JP); FUJII, Takaaki, Tokyo, 100-8280 (JP); UMEKAWA, Toru, Tokyo, 105-6409 (JP); NAKAMURA, Mitsuhiro, Kyoto, 606-8507 (JP); MIZOWAKI, Takashi, Kyoto, 606-8501 (JP); KISHI, Noriko, Kyoto, 606-8501 (JP); SHIMIZU, Yukine, Kyoto, 606-8507 (JP); SAKURAI, Yuta, Tokyo, 105-6409 (JP)
(74) Representative: MERH-IP Matias Erny Reichl Hoffmann Patentanwälte PartG mbB

(57) **Abstract**

A moving object tracking calculation device evaluates priority of visibility of a tracking target between a high-visibility digital radiography image and a low-visibility digital radiography image of the tracking target, calculates a weight distribution representing relative presence accuracy of the tracking target in the low-visibility digital radiography image using epipolar geometry and a difference in visibility between the high-visibility digital radiography image and the low-visibility digital radiography image, and determines a detection position of the tracking target in the low-visibility digital radiography image using the weight distribution and a similarity distribution of the low-visibility digital radiography image.

## Description

### BACKGROUND OF THE INVENTION

### 1. Field of the Invention

The present invention relates to a tracking target position detection device, a radiotherapy system, and a tracking target position detection method suitable for a radiotherapy system that treats an affected area (hereinafter, referred to as a "target") such as a tumor by irradiating the affected area with radiation such as charged particles or X-rays, and a moving object tracking device used in the radiotherapy system.

### 2. Description of Related Art

As an example of a system for tracking a target that moves during radiation therapy, PTL 1 discloses that a target is directly tracked during image guided radiation therapy using a 2D contour and a search area.

As an example of a radiation irradiation system and a moving object tracking device capable of accurately detecting a tracking target even when an imaging condition by X-rays is severe, such as when a subject is thick, PTL 2 discloses that template matching is performed on two digital radiography images using a template image representing a fiducial marker prepared in advance, positions having a high matching score are listed as candidates for a fiducial marker position, a length of a common perpendicular line is calculated for all combinations from two candidate lists of the fiducial marker positions, a position of the fiducial marker is detected based on the matching score and the common perpendicular line, and extraction of a proton beam with which a target is irradiated is controlled based on the detected position of the fiducial marker.

### Citation List

### Patent Literature

PTL 1: US7334590
PTL 2: JP2017-209243A

### SUMMARY OF THE INVENTION

A moving body tracking radiotherapy system is known in which a position of a target is recognized in real time and therapeutic radiation is emitted to concentrate the therapeutic radiation on the target and reduce irradiation of organs around the target.

As a unit that recognizes a target position, an image recognition unit that captures X-ray images from a plurality of directions and recognizes the target position from the captured images is used.

As a Method of recognizing the target position, there is a method of directly recognizing the target, and a method of indirectly recognizing the target from a tracking target such as a landmark in a body such as a lateral diaphragm or an alternative fiducial marker inserted near the target.

In any case, a projection image of the target or the tracking target is prepared in advance as a template image, and a two-dimensional position of the target is calculated by performing matching processing (template matching) for searching for a place most similar to the template image in the captured image. Then, a three-dimensional position of the tracking target is calculated based on the two-dimensional position of the target obtained from the captured image in each direction and geometric information on an imaging system.

When X-ray fluoroscopy from two directions is used, a position at which two lines connecting a position on an X-ray measurement device where the tracking target is imaged and an X-ray generator for fluoroscopy are closest to each other is regarded as a position of the tracking target. At the position at which the two lines are closest to each other, a line perpendicular to the two lines can be drawn. This vertical line is referred to as a common perpendicular line, and a midpoint of the common perpendicular line is regarded as the position of the tracking target.

When the target is directly tracked, a three-dimensional position of the tumor can be directly recognized, but when the landmark in the body or the alternative fiducial marker is used as the tracking target, the target position is indirectly recognized by calculating a relationship between the three-dimensional position of the tracking target and the three-dimensional position of the target in advance.

By controlling the irradiation of the therapeutic radiation by recognizing the position of the target, it is possible to accurately irradiate targets in organs that move during treatment, such as respiratory moving organs.

Here, due to a difference in a method of controlling the irradiation of the therapeutic radiation, two irradiation control methods have been proposed, that is, a gating irradiation in which the irradiation is performed at a timing when the target position and an radiation irradiation position according to a treatment plan coincide with each other, and a dynamic tumor-tracking irradiation in which the radiation irradiation position is changed according to the target position.

In any irradiation control method, it is important to determine success or failure of position recognition of the tracking target to implement highly accurate irradiation.

In an image recognition method represented by template matching, a similarity scale, such as zero-mean normalized cross-correlation (ZNCC), is calculated using the template image and an X-ray image, a position at which the similarity scale is the largest and the X-ray image is most similar to the template image is searched for, and this position is set as a recognition position of the tracking target.

In contrast, in a digital radiography image on one side, when the imaging condition by X-rays is severe, such as when the subject is thick, and a structure similar to the tracking target appears in the vicinity of the tracking target on the digital radiography image, a non-tracking target may be erroneously detected as the tracking target. In this case, a distance between the two lines connecting the position on the X-ray measurement device where the tracking target is imaged and the X-ray generator for fluoroscopy increases, whereby a length of the common perpendicular line of the tracking target increases, and position recognition accuracy of the tracking target decreases.

In contrast, in PTL 1 described above, a method of limiting a length in a longitudinal direction of the search area used for calculating the similarity scale with the tumor in a second digital radiography image based on a detection position of the tumor in a first digital radiography image, has been reported.

The detection position of the tracking target in each of a pair of digital radiography images has uncertainty in the detection position. Therefore, robustness of the detection position can be improved by modulating a strength of the limit of the search area according to a difference in visibility of the tumor in each digital radiography image in the pair of digital radiography images. However, even in the method of PTL 1, it is difficult to provide different levels of limits on the search area.

In contrast, in PTL 2, to improve recognition accuracy of the three-dimensional position of the tracking target, a method has been developed in which a value of the similarity scale and a common perpendicular line length are obtained, a value indicating certainty of the detection and the similarity scale are weighted, and the position of the tracking target is detected based on a result of the weighting.

However, as a result of intensive studies by the present inventors, it has been found that when the method of PTL 2 is applied to direct tracking of the target or the landmark in a large body such as a lateral diaphragm, the number of combinations of detection candidates for each digital radiography image for calculating the common perpendicular line length increases, and therefore, there is room for improvement in determining erroneous detection in a short time.

The invention provides a tracking target position detection device, a radiotherapy system, and a tracking target position detection method that achieve both further improvement in accuracy of detection of a tracking target and reduction in calculation time.

The invention includes a plurality of units for solving the above problems, and examples thereof include a device for detecting a position of a tracking target. The device includes: one or more sets of digital radiography devices including a fluoroscopy radiation detector and a fluoroscopy radiation generation device as one set and configured to capture a digital radiography image including the tracking target; and a calculation device configured to obtain the position of the tracking target from the digital radiography image obtained by the digital radiography device, in which the calculation device evaluates priority of visibility of the tracking target among a plurality of the digital radiography images of the tracking target, calculates a weight distribution representing relative presence accuracy of the tracking target in a low-visibility digital radiography image using epipolar geometry and a difference in the visibility among the digital radiography images, and determines a detection position of the tracking target in the low-visibility digital radiography image using the weight distribution and a similarity distribution of the low-visibility digital radiography image.

According to the invention, it is possible to achieve both further improvement in accuracy of detection of a tracking target and reduction in calculation time. Problems, configurations, and effects other than those described above will be clarified by the following description of the embodiment.

### BRIEF DESCRIPTION OF THE DRAWINGS

FIG. 1 is an overall configuration diagram of an X-ray therapy system including a moving object tracking device according to an embodiment;
FIG. 2 is a flowchart illustrating a calculation process of a recognition position of a tracking target in the moving object tracking device according to the embodiment;
FIG. 3 is a flowchart illustrating a calculation process of a detection position in a low-visibility digital radiography image in the moving object tracking device according to the embodiment;
FIG. 4 is a diagram relating to a setting of a high accuracy region in the low-visibility digital radiography image in the moving object tracking device according to the embodiment;
FIG. 5 is a diagram relating to a calculation method of a weight map in the low-visibility digital radiography image in the moving object tracking device according to the embodiment;
FIG. 6 is a diagram relating to the calculation method of the weight map in the low-visibility digital radiography image in the moving object tracking device according to the embodiment; and
FIG. 7 is a diagram illustrating an embodiment of a display screen of a calculation result of a detection position in the moving object tracking device according to the embodiment.

### DESCRIPTION OF EMBODIMENTS

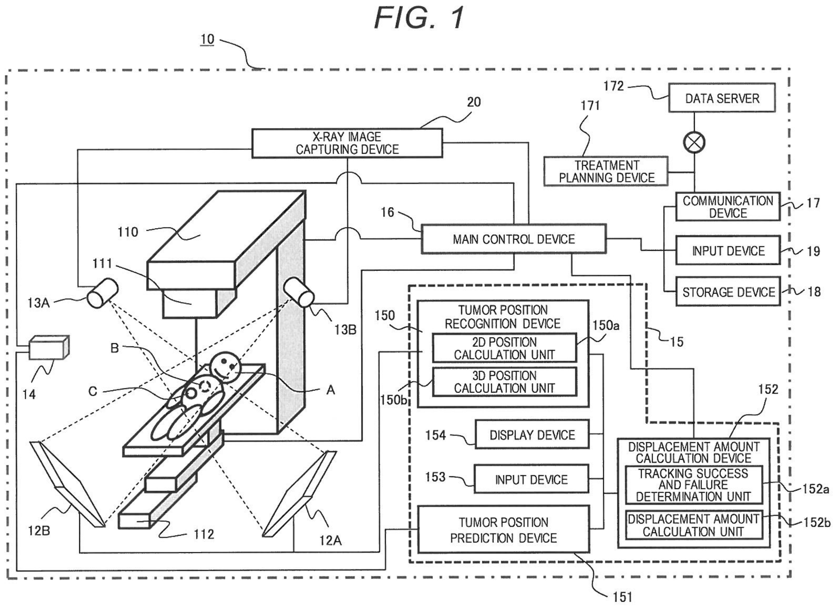

An embodiment of a tracking target position detection device, a radiotherapy system, and a tracking target position detection method according to the invention will be described with reference to FIGS. 1 to 7.

In the drawings used in the present description, the same or corresponding components are denoted by the same or similar reference signs, and repeated descriptions of these components may be omitted.

In the following embodiment, a case in which the invention is applied to an X-ray therapy system using X-rays as radiation will be described, but the radiation to be used is not limited to X-rays, and particle beams of protons, helium, carbon, or the like can be used.

A mode (dynamic tumor-tracking irradiation) in which a position of a moving object is tracked and a target is irradiated with the radiation while changing an irradiation position of the radiation with respect to the target will be described, but the invention can also be applied to a mode (gating irradiation) in which the position of the moving object is tracked and the target is irradiated with the radiation only when the target is present in a predetermined region.

First, an overall configuration of the X-ray therapy system according to the invention will be described with reference to FIG. 1. FIG. 1 is an overall configuration diagram of the X-ray therapy system including a moving object tracking device according to an embodiment.

An X-ray therapy system 10 illustrated in FIG. 1 is a radiotherapy system including a device group for irradiating a tumor present in a patient A who is a subject with X-rays as the target. The X-ray therapy system 10 illustrated in FIG. 1 includes an X-ray irradiation device, X-ray detectors 12A and 12B, X-ray tubes 13A and 13B, an infrared camera 14, a moving object tracking calculation device 15, a main control device 16, a communication device 17, a storage device 18, an input device 19, and an X-ray image capturing device 20.

Among these, the X-ray detectors 12A and 12B and the X-ray tubes 13A and 13B constitute one or more sets of digital radiography devices that capture digital radiography images of a tracking target B. Although the therapy system according to the present embodiment includes two sets of digital radiography devices, the number of sets of digital radiography devices may be three or more and is not particularly limited.

The X-ray detectors 12A and 12B, the X-ray tubes 13A and 13B, and the moving object tracking calculation device 15 constitute the moving object tracking device.

The X-ray irradiation device includes a gantry 110, an irradiation nozzle 111 that extracts radiation for treatment, and a couch 112.

When an extraction start signal is output from the main control device 16, a linear accelerator in the irradiation nozzle 111 accelerates an electron beam and irradiates tungsten with the accelerated electron beam to generate X-rays.

A collimator (to be referred to as a multi-leaf collimator ((MLC) hereinafter) including a plurality of plate-shaped shields (to be referred to as leaf hereinafter) disposed on the left and right sides is provided in the irradiation nozzle 111, and the generated X-rays are shaped into a desired distribution by freely changing a position of each leaf based on an instruction value from the main control device 16. A dose monitor that measures an X-ray irradiation amount is further provided in the irradiation nozzle 111, and a detected measurement value is output to the main control device 16 and used for control during radiation irradiation.

The irradiation nozzle 111 is mounted on the gantry 110, receives the instruction value from the main control device 16, sets a gantry rotation angle, and can irradiate the patient A on the couch 112 with X-rays from any angle. A rotation center of the gantry 110 is called an isocenter. The irradiation nozzle 111 includes a gimbal mechanism that swings the irradiation nozzle 111 in a lateral direction (x direction and y direction) to track a respiratory moving organ.

The main control device 16 is connected to the gantry 110, the irradiation nozzle 111, the couch 112, the moving object tracking calculation device 15, the communication device 17, the storage device 18, the input device 19, and the X-ray image capturing device 20, and controls the devices in the irradiation nozzle 111, the gantry 110, the couch 112, and the X-ray image capturing device 20.

The communication device 17 is connected to a data server 172 via a network, acquires, from the data server 172, irradiation parameters (gantry angle, irradiation amount, leaf position information, and the like) created by a treatment planning device 171 via the network before the irradiation, and stores the irradiation parameters in the storage device 18.

The input device 19 is connected to the main control device 16, receives an input signal from a medical worker who operates the X-ray therapy system 10, and transmits various control signals to the main control device 16. Upon receiving an irradiation start instruction of the radiation via the input device 19, the main control device 16 starts the irradiation based on the irradiation parameters stored in the storage device 18.

The X-ray therapy system 10 includes the X-ray tube 13A that irradiates the patient A with the X-rays from a first direction, the X-ray detector 12A that detects a two-dimensional dose distribution of the X-rays emitted from the X-ray tube 13A and transmitted through the patient A, and a signal processing circuit (not illustrated).

The X-ray detector 12A includes a plurality of two-dimensionally arranged detection elements (specifically, for example, semiconductor elements that convert the radiation into electric charges), and outputs analog signals from the detection elements. The signal processing circuit processes the analog signals from the X-ray detector 12A to generate data of an X-ray image, and transmits the data to a tumor position recognition device 150.

Imaging by an X-ray digital radiography device is performed at a frequency (for example, about 1 Hz to 2 Hz) sufficient to confirm movement of the tracking target B.

Meanwhile, imaging by the infrared camera 14 is performed at a frequency (for example, about 60 Hz) at which movement of a body surface by an infrared fiducial marker C can be captured with sufficient accuracy.

Similarly, the X-ray therapy system 10 includes the X-ray tube 13B that irradiates the patient A with the X-rays from a second direction (in the present embodiment, a direction orthogonal to the first direction), the X-ray detector 12B that detects a two-dimensional dose distribution of the X-rays emitted from the X-ray tube 13B and transmitted through the patient A, and a signal processing circuit (not illustrated). The X-ray detector 12B includes a plurality of two-dimensionally arranged detection elements, and outputs analog signals from the detection elements. The signal processing circuit processes the analog signals from the X-ray detector 12B to generate data of an X-ray image, and transmits the data to the tumor position recognition device 150. Imaging by the X-ray tube 13B is performed in synchronization with the imaging by the X-ray tube 13A.

Here, X-ray emission intervals of the X-ray tubes 13A and 13B, as well as imaging parameters such as tube voltage and tube current, are set by the X-ray image capturing device 20 via the input device 19.

The X-ray image capturing device 20 controls the irradiation of the X-rays for transmission based on a signal from the main control device 16.

The moving object tracking calculation device 15 is a device that executes a program to be described later and obtains a position of the tracking target B, and includes the tumor position recognition device 150, a tumor position prediction device 151, a displacement amount calculation device 152, an input device 153 that provides input information to each device, and a display device 154 that displays an output from each device.

The tumor position recognition device 150 is a calculation processing device that recognizes the position of the tracking target B in real time from images captured using the X-ray tubes 13A and 13B that perform X-ray imaging of the tracking target B in the patient A on the couch 112 from a plurality of directions and calculates a tumor position, and includes a 2D position calculation unit 150a and a 3D position calculation unit 150b.

The 2D position calculation unit 150a calculates a recognition position and a recognition candidate position of the tracking target B in each digital radiography image acquired by the X-ray detector 12A and the X-ray detector 12B.

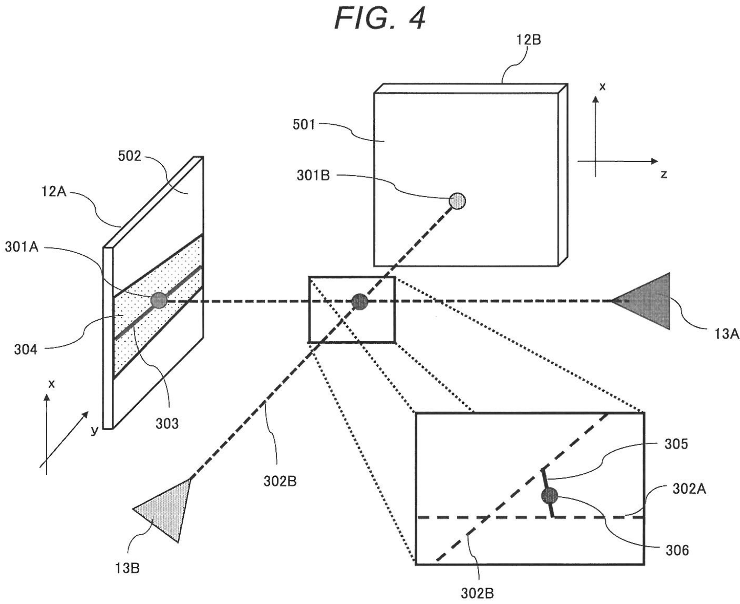

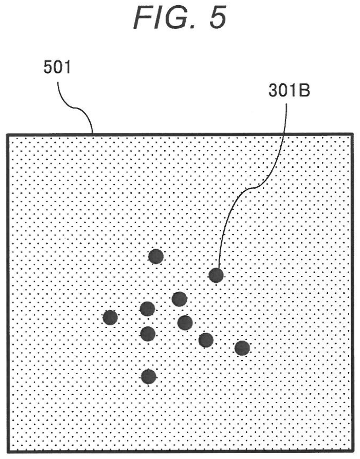

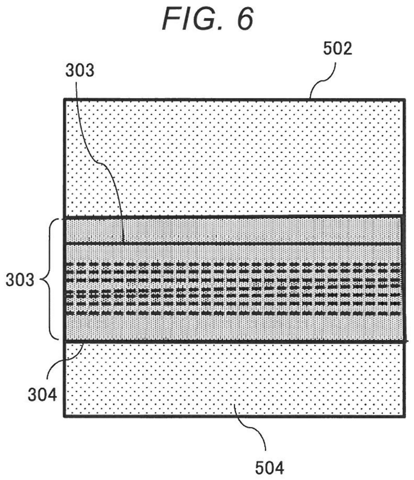

More specifically, the 2D position calculation unit 150a evaluates visibility of the tracking target B between a high-visibility digital radiography image 501 (see FIGS. 4 and 5) and a low-visibility digital radiography image 502 (see FIGS. 4 and 6) of the tracking target B to determine a priority, calculates a weight distribution (weight map) representing relative presence accuracy of the tracking target B in the low-visibility digital radiography image 502 by using epipolar geometry and a difference in visibility between the high-visibility digital radiography image 501 and the low-visibility digital radiography image 502, and calculates the recognition position and the recognition candidate position of the tracking target B using the weight distribution and a similarity distribution of the low-visibility digital radiography image 502, that is, determines a detection position of the tracking target B.

A calculation process of the recognition position and the recognition candidate position of the tracking target B in the low-visibility digital radiography image 502 performed by the 2D position calculation unit 150a is the largest feature of the invention, and recognition accuracy of the position of the tracking target B in the low-visibility digital radiography image 502 can be efficiently improved.

The 2D position calculation unit 150a is preferably an execution subject of an evaluation step of evaluating the priority of the visibility of the tracking target B between the high-visibility digital radiography image 501 and the low-visibility digital radiography image 502 of the tracking target B, a calculation step of calculating the weight distribution representing the relative presence accuracy of the tracking target B in the low-visibility digital radiography image 502 by using the epipolar geometry and the difference in visibility between the high-visibility digital radiography image 501 and the low-visibility digital radiography image 502, and a determination step of determining the detection position of the tracking target B in the low-visibility digital radiography image 502 using the weight distribution and the similarity distribution of the low-visibility digital radiography image 502.

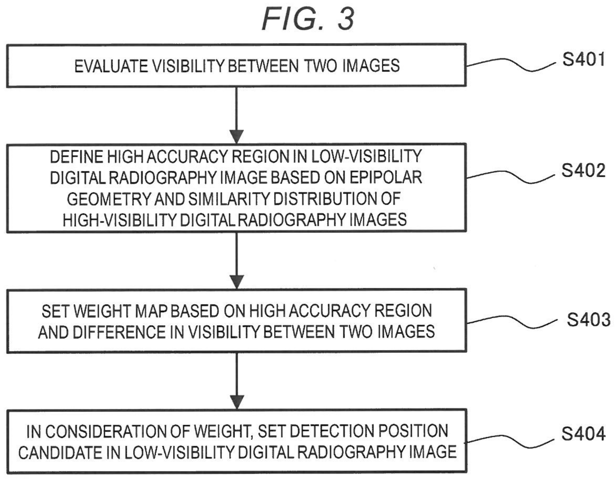

In FIG. 3 to be described later, step S401 corresponds to the evaluation step, step S402 and step S403 correspond to the calculation step, and step S404 corresponds to the determination step.

The 3D position calculation unit 150b calculates three-dimensional coordinates of the tumor based on the detection position of the tracking target B or a detection candidate in each digital radiography image calculated by the 2D position calculation unit 150a.

The tumor position prediction device 151 recognizes position information on the body surface from the image of the infrared fiducial marker C provided on the body surface of the patient A, which is captured by the infrared camera 14, and predicts the tumor position from a body surface position using a correlation model between the body surface and the tumor position.

The displacement amount calculation device 152 is a calculation processing device that calculates, based on a tumor position recognition result obtained by the tumor position recognition device 150 and a tumor position prediction result obtained by the tumor position prediction device 151, a displacement amount of a target position from an image capturing timing before the tracking target B and a displacement amount of the gimbal mechanism for irradiating the target moved by the displacement amount with the X-rays, and includes a tracking success and failure determination unit 152a and a displacement amount calculation unit 152b.

The tracking success and failure determination unit 152a compares the prediction result of the tumor position calculated by the tumor position prediction device 151 with the tumor position recognition result of the tumor position recognition device 150, and determines the success or failure of tracking. The displacement amount calculation unit 152b calculates the displacement amount of the gimbal mechanism based on the prediction result of the tumor position.

In X-ray dynamic tumor-tracking irradiation as in the present embodiment, since a gimbal is moved according to the displacement amount of the target position, the displacement amount calculation unit 152b obtains the displacement amount of the target position and the displacement amount of the gimbal mechanism, but in particle beam irradiation, for example, on since on and off control of irradiation of particle beams is executed, the displacement amount calculation unit 152b calculates the displacement amount of the target position.

Each of the main control device 16, the moving object tracking calculation device 15, and the X-ray image capturing device 20 may have a configuration including a central processing unit (CPU) and a memory connected to the CPU, or these devices may be implemented by an integrated computer. The invention is not particularly limited thereto.

Control processing on operations to be executed may be integrated into one program, may be divided into a plurality of separate programs, or may be a combination thereof.

A part or all of the programs held by each device may be implemented by dedicated hardware or may be modularized. Various programs may be installed in each device by a program distribution server or an external storage medium, or an existing device may be updated.

Each device may be an independent device connected by a wired or wireless network, or two or more devices may be integrated.

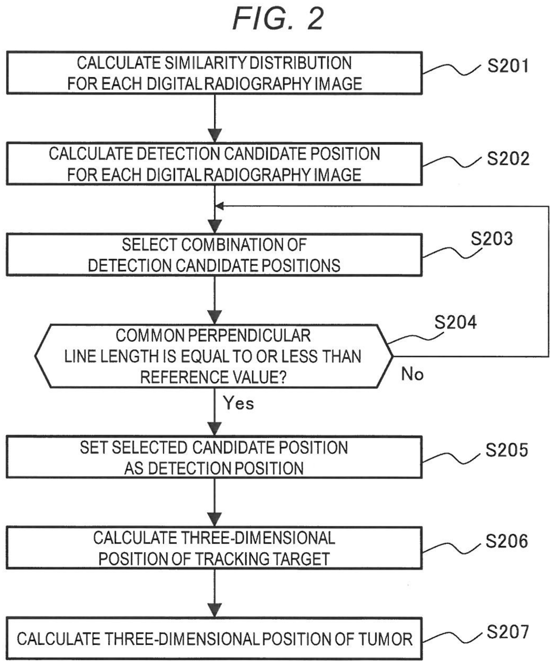

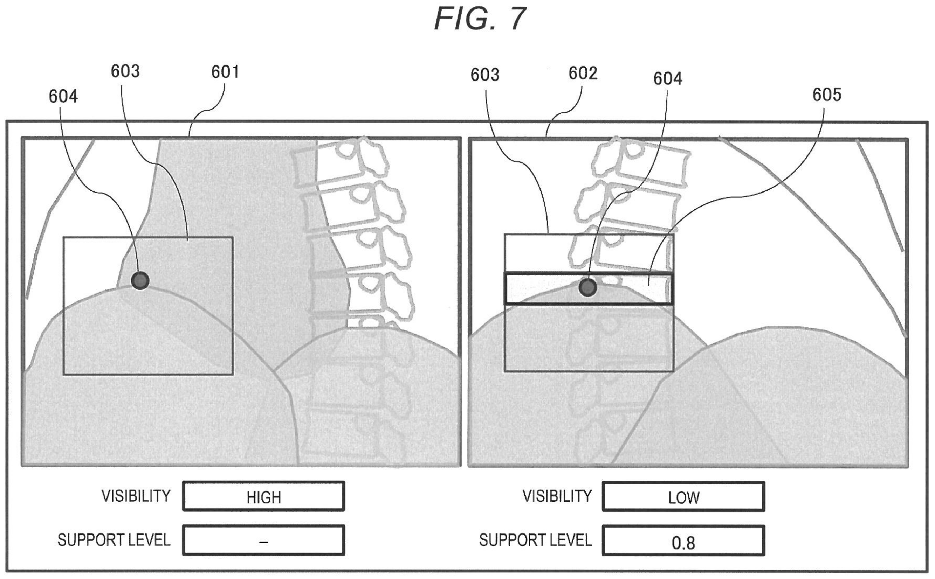

Next, a recognition process of the tumor position, which is a feature of the invention, is also a detection method of the position of the tracking target B, and is preferably performed by the tumor position recognition device 150 at the time of performing moving object tracking will be described with reference to FIGS. 2 to 7. FIG. 2 is a flowchart illustrating the calculation process of the recognition position of the tracking target B. FIG. 3 is a flowchart illustrating a calculation process of the detection position in the low-visibility digital radiography image. FIG. 4 is a diagram relating to a setting of a high accuracy region in the low-visibility digital radiography image. FIGS. 5 and 6 are diagrams relating to a calculation method of a weight map in the low-visibility digital radiography image. FIG. 7 is a diagram illustrating an example of a display screen of a calculation result of the detection position.

First, as illustrated in FIG. 2, the 2D position calculation unit' 150a of the tumor position recognition device 150 calculates the similarity distribution based on a template image and each digital radiography image acquired by the X-ray detector 12A and the X-ray detector 12B (step S201).

The zero-mean normalized cross-correlation (ZNCC) can be used as the similarity scale in step S201. In this case, the similarity distribution ZNCC (X,Y) is calculated by the following formula (1). Here, the similarity distribution is a distribution of similarities between the template image and a part of the digital radiography image, and a method called a sum of squared differences (SSD) or a sum of absolute differences (SAD) is used in addition to the zero-mean normalized cross-correlation (ZNCC).
Math. 1 $ZNCC \left(X, Y\right) = \frac{{\sum }_{j = 0}^{N - 1} {\sum }_{i = 0}^{M - 1} \left(I\left(X-M/2+i,Y-N/2+j\right)-\overline{I}\right) \left(T\left(i, j\right)-\overline{T}\right)}{\sqrt{{\sum }_{j = 0}^{N - 1} {\sum }_{i = 0}^{M - 1} {\left(I\left(X-M/2+i,Y-N/2+j\right)-\overline{I}\right)}^{2} \times {\sum }_{j = 0}^{N - 1} {\sum }_{i = 0}^{M - 1} {\left(T\left(i, j\right)-\overline{T}\right)}^{2}}}$

Here, i and j in Formula (1) represent a horizontal index and a vertical index of an image, and M and N represent the numbers of pixels in a lateral direction and a vertical direction of the template image. I(i,j) represents a pixel value of the digital radiography image, T(i,j) represents a pixel value of the template image, I and T are averages in a region of interest (template image size) of the digital radiography image and the template image, respectively, which are expressed by the following formula (2).
Math. 2 $\overline{I} = \frac{1}{MN} {\sum }_{j = 0}^{N - 1} {\sum }_{i = 0}^{M - 1} I \left(X-M/2+i,Y-N/2+j\right) , \overline{T} = \frac{1}{MN} {\sum }_{j = 0}^{N - 1} {\sum }_{i = 0}^{M - 1} T \left(i, j\right)$

In this way, the higher the similarity scale is, the more similar a part of the template image and the digital radiography image is, and the more the template image is recognized. Therefore, the similarity scale can be used to evaluate the priority of the visibility of the template in the digital radiography image by a magnitude of a numerical value.

Subsequently, the 2D position calculation unit 150a calculates a detection candidate for the tracking target B in each of the digital radiography images acquired by the X-ray detector 12A and the X-ray detector 12B for each digital radiography image (step S202). Details of step S202 will be described with reference to a flow illustrated in FIG. 3. The details of step S202 are illustrated in FIG. 3.

First, as illustrated in FIG. 3, the visibility between two digital radiography images is evaluated, and the priority is given to the visibility of a pair of digital radiography images (step S401). In this way, in the evaluation step, the visibility of the tracking target B can be determined based on the similarity distributions of the high-visibility digital radiography image 501 and the low-visibility digital radiography image 502.

In step S401, an average value of the similarity scale at an upper position of the similarity distribution or a gradient information on the similarity distribution at the upper position can be used to evaluate the visibility. An operator can determine and designate the priority of the visibility of each digital radiography image for each irradiation angle range in advance using a virtual digital radiography image (digital reconstructed radiography (DRR)) simulated based on a CT image of the patient in advance.

Subsequently, a high accuracy region 304 is defined in the low-visibility digital radiography image 502 based on the epipolar geometry and a similarity distribution of the high-visibility digital radiography image 501 (step S402). A method of defining the high accuracy region 304 in step S402 will be described with reference to FIGS. 4 to 6.

In FIG. 4, a case in which the image acquired by the X-ray detector 12B is the high-visibility digital radiography image 501 and the image acquired by the X-ray detector 12A is the low-visibility digital radiography image 502 will be described as an example.

First, the 2D position calculation unit 150a sets a position having a higher similarity scale as the detection candidate for the high-visibility digital radiography image 501 from the similarity distribution of the high-visibility digital radiography image 501.

As illustrated in FIG. 5, when one point among the detection candidates in the high-visibility digital radiography image 501 is set as a tracking candidate position 301B, it is considered that there is a high possibility that a tracking candidate position 301A indicating the tracking target B is present on a straight line 302B connecting the tracking candidate position 301B and the X-ray tube 13B. Therefore, on the low-visibility digital radiography image 502, there is a high possibility that the tracking target B is present on a straight line obtained by projecting the straight line 302B from the X-ray tube 13B to the X-ray detector 12A. This straight line is referred to as an epipolar line 303.

Therefore, when the epipolar line 303 is drawn on the low-visibility digital radiography image 502 with respect to all the tracking candidate positions 301B in the high-visibility digital radiography image 501, the high accuracy region 304 surrounded by a plurality of the epipolar lines 303 is formed as illustrated in FIGS. 4 and 6. Since this region is considered to be a region in which presence accuracy of the tracking target B is relatively high in the low-visibility digital radiography image 502, this region is defined as the high accuracy region 304.

Subsequently, a weight distribution W (X,Y) representing relative presence accuracy of the tracking target B is generated based on the high accuracy region 304 and a difference in visibility between the two digital radiography images (step S403). Here, a case in which the weight distribution is generated by determination inside and outside the high accuracy region 304 will be described as an example. When the high accuracy region 304 is A_{high}, the weight distribution at each position (X,Y) of the low-visibility digital radiography image 502 is defined by the following formula (3).
Math. 3 $\left\{\begin{matrix}w \left(X, Y\right) = 1.0 , & X , Y ∈ {A}_{high} \\ w \left(X, Y\right) = 1.0 - \left(\overline{{S}_{high}}-\overline{{S}_{low}}\right) \times m & X , Y ∈ {A}_{high}^{C}\end{matrix}\right.$

Here, A_{high}^{C} represents an outside of the high accuracy region 304, S_{high} and S_{low} represent the visibility of the high-visibility digital radiography image 501 and the low-visibility digital radiography image 502 calculated in step S401, and m represents an adjustment coefficient.

In the high accuracy region 304, 1.0 is set as an accuracy weight, while outside the high accuracy region 304, the accuracy weight that decreases according to a difference in average values of the similarity scales between the images is set.

In this way, in the calculation step, the weight distribution can be reduced in a region 504 outside the high accuracy region 304 surrounded by the epipolar lines based on the upper position of the similarity distribution of the high-visibility digital radiography image 501.

By using Formula (3), as the difference in visibility between the digital radiography images increases, a value of the accuracy weight inside and outside the high accuracy region 304 can be made different. Accordingly, it is possible to automatically adjust a strength of assistance of the epipolar geometry according to the difference in visibility, and it is possible to place more importance on a tracking result of the high-visibility digital radiography image 501 as the difference in visibility increases, while placing more importance on the similarity distribution in the low-visibility digital radiography image 502 when the difference in visibility is small.

Although an example in which the weight in the high accuracy region 304 is constant has been described so far, when the high accuracy region 304 is calculated in step S402, a distribution in which the weight in the high accuracy region 304 is increased or decreased may be provided according to a density of the used epipolar lines or a magnitude of the similarity scale of the detection candidate of the high-visibility digital radiography image 501.

When the visibility is close between the two digital radiography images, regardless of a relative value of the visibility, a difference between S_{high} and S_{low} in Formula (3) is small, and the weights inside and outside the high accuracy region 304 are substantially the same.

Although Formula (3) indicates a case in which the accuracy weight decreases according to the difference in average value of the similarity scales between the images outside the high accuracy region 304, conversely, it is also possible to increase the accuracy weight according to the difference in average value of the similarity scales between the images in the high accuracy region 304.

Subsequently, in the low-visibility digital radiography image 502, template matching is performed in consideration of a weight distribution w (X,Y) representing a relative presence accuracy distribution of the tracking target (step S404). A modified similarity distribution ZNCC_{mod} (X,Y) considering the weight distribution is defined by the following formula (4).
Math. 4 ${ZNCC}_{mod} \left(X, Y\right) = ZNCC \left(X, Y\right) \times w \left(X, Y\right)$

A position having a higher modified similarity scale, which is a similarity scale considering a weight, is stored as the detection candidate of the low-visibility digital radiography image 502 (step S404).

In this way, by using the modified similarity distribution obtained by multiplying the similarity distribution ZNCC (X,Y) by the weight distribution w (X,Y), when the difference in visibility between the two digital radiography images is large, the value of the similarity scale in the region outside the high accuracy region 304 can be significantly reduced, and the points in the high accuracy region 304 can be selectively ranked. In contrast, when the difference in visibility is small, it is possible to calculate the detection candidate for the low-visibility digital radiography image 502 by placing importance on the magnitude of the similarity distribution of the low-visibility digital radiography image 502. Accordingly, it is possible to calculate more robust detection candidates according to the visibility between the two images.

Returning to FIG. 2, when the calculation of the detection candidate in each digital radiography image is completed, the 3D position calculation unit 150b of the tumor position recognition device 150 selects a set of tracking candidate positions in order from candidates having a larger similarity scale or a higher modified similarity scale from the tracking candidate positions of the high-visibility digital radiography image 501 and the low-visibility digital radiography image 502 (step S203).

Subsequently, the 3D position calculation unit 150b calculates a common perpendicular line 305 between the straight line 302A connecting the tracking candidate position 301B of the high-visibility digital radiography image 501 and the X-ray tube 13B and the straight line 302B connecting the tracking candidate position 301A of the low-visibility digital radiography image 502 and the X-ray tube 13A. Then, it is determined whether a length of the calculated common perpendicular line 305 is equal to or less than a reference value (step S204), and if the length is equal to or less than the reference value, the selected detection candidate is set as the detection position, that is, as a three-dimensional position 306 of the tracking target B (step S205). On the other hand, if the length of the common perpendicular line 305 exceeds the reference value, the processing returns to step S203 to select a different set of detection candidates. Here, a common perpendicular line length is used to determine the detection position, but a common perpendicular line length weighted by the value of the modified similarity scale may be used.

Subsequently, the 3D position calculation unit 150b calculates a midpoint of the common perpendicular line 305 of the straight line connecting the detection position of each digital radiography image and each of the X-ray tubes 13A and 13B as the three-dimensional position 306 of the tracking target B (Step S206).

By using the calculation method of the detection candidate in the low-visibility digital radiography image 502, which is a feature of the invention, the points in the high accuracy region 304 in the low-visibility digital radiography image 502 are higher in the modified similarity scale and are preferentially selected as the detection candidate, and therefore, the number of iterations of the determination of the common perpendicular line length is reduced, and a calculation time until the three-dimensional position of the tracking target B is calculated can be reduced.

Subsequently, the 3D position calculation unit 150b calculates the three-dimensional position of the tumor based on the three-dimensional position of the tracking target B (step S207). When the tracking target B is a tumor, the three-dimensional position of the tracking target B becomes the three-dimensional position of the tumor as it is, but when the tracking target B is a substitute fiducial marker such as a lateral diaphragm or a gold fiducial marker, the relationship (offset vector) between the three-dimensional position of the tracking target B and the three-dimensional position of the tumor calculated in advance is read to calculate the coordinates of the target position.

A calculation flow of the three-dimensional position of the tumor illustrated in FIG. 2 is based on the premise that a plurality of detection candidates are held in each digital radiography image. However, it is not always necessary to hold a plurality of candidate positions for each digital radiography image.

For example, only a detection candidate having the highest similarity scale and modified similarity scale among the detection candidates in each digital radiography image may be held as the detection position, and step S203 and step S204 may be omitted.

The calculation process of the three-dimensional position of the target described so far is used in a process of generating and updating the correlation model between the body surface and the tumor position used by the tumor position prediction device 151 before or during irradiation of the beam in the moving object dynamic tumor-tracking irradiation, and a process in which the tracking success and failure determination unit 152a of the displacement amount calculation device 152 confirms the validity of the tumor position predicted by the tumor position prediction device 151 using the correlation model between the body surface and the tumor position at the time of irradiation and determines the tracking success and failure.

A difference in visibility between the high-visibility digital radiography image 501 and the low-visibility digital radiography image 502, the weight distribution thereof, and the calculation result of the detection position in the high-visibility digital radiography image 501 or the low-visibility digital radiography image 502, which are features of the invention, can be displayed on the display device 154.

For example, as illustrated in FIG. 7, a search area 603 and a detection position 604 of the tracking target B are displayed in each of a high-visibility digital radiography image 601 and a low-visibility digital radiography image 602, and a weight distribution 605 is displayed in the low-visibility digital radiography image 602.

It is also possible to clearly show the difference in visibility between the two images to the operator, such as displaying only a minimum value of the weight distribution or displaying a level of assistance (large assistance, medium assistance, small assistance) by the epipolar geometry according to the magnitude of the minimum value.

A calculation method of the detection candidate in the low-visibility digital radiography image 502, which is a feature of the invention, is also applicable to the calculation process of the position of the tracking target B in the gating irradiation.

In the gating irradiation, a three-dimensional gate region is set, a three-dimensional position of the tracking target B is calculated, and it is determined whether the tracking target B is present in the gate region, thereby determining whether the beam extraction is possible. In general, in the gating irradiation, unlike the dynamic tumor-tracking irradiation, a digital radiography image is captured frequently, and ON/OFF of the radiation irradiation is often determined only based on the position of the tracking target B calculated based on the digital radiography image.

Although an X-ray therapy device has been described as an example so far, the invention can also be applied to a particle beam therapy device that accelerates and irradiates a proton beam or a carbon beam using an accelerator.

Next, effects of the present embodiment will be described.

The device for detecting the position of the tracking target B according to the present embodiment described above includes one or more sets of digital radiography devices that include the X-ray detectors 12A and 12B and the X-ray tubes 13A and 13B as one set and capture the high-visibility digital radiography image 501 and the low-visibility digital radiography image 502 including the tracking target B, and the moving object tracking calculation device 15 that obtains the position of the tracking target B based on the high-visibility digital radiography image 501 and the low-visibility digital radiography image 502 obtained by the digital radiography devices. The moving object tracking calculation device 15 evaluates the priority of the visibility of the tracking target B between the high-visibility digital radiography image 501 and the low-visibility digital radiography image 502 of the tracking target B, calculates the weight distribution representing the relative presence accuracy of the tracking target B in the low-visibility digital radiography image 502 using the epipolar geometry and the difference in visibility between the high-visibility digital radiography image 501 and the low-visibility digital radiography image 502, and determines the detection position of the tracking target B in the low-visibility digital radiography image 502 using the weight distribution and the similarity distribution of the low-visibility digital radiography image 502.

Accordingly, even when the visibility of the tracking target B is reduced in one digital radiography image of the pair of digital radiography images, the position detection accuracy of the tracking target B can be efficiently improved in the low-visibility digital radiography image 502 by using the result of the high-visibility digital radiography image 501, and therefore, the detection accuracy of the tracking target B can be further improved while reducing the calculation time as compared with the related art, and the reliability of the gating irradiation and the dynamic tumor-tracking irradiation can be improved.

Since the moving object tracking calculation device 15 reduces the weight distribution in the region outside the high accuracy region 304 surrounded by the epipolar lines based on the upper position of the similarity distribution of the high-visibility digital radiography image 501, it is possible to further improve the detection accuracy of the tracking target B in the low-visibility digital radiography image 502.

By displaying the calculation result of the detection position in the low-visibility digital radiography image 602 on the display device 154 by using the difference in visibility between the high-visibility digital radiography image 601 and the low-visibility digital radiography image 602 and the weight distribution, the operator can confirm whether a point in a region (high accuracy region 304) in which the weight distribution 605 is high is selected as the detection position in the low-visibility digital radiography image 602, and how much the assistance strength by the epipolar geometry is.

The moving object tracking calculation device 15 determines the visibility of the tracking target B based on the similarity distribution of each of the high-visibility digital radiography image 501 and the low-visibility digital radiography image 502, and therefore, it is possible to stably determine the priority of the visibility with high accuracy.

### Others

The invention is not limited to the embodiment described above, and various modifications and applications are possible. The above-described embodiment is described in detail for easy understanding of the invention, and the invention is not necessarily limited to those having all the configurations described above.

## Claims

1. A tracking target position detection device for detecting a position of a tracking target, the tracking target position detection device comprising:
one or more sets of digital radiography devices including a fluoroscopy radiation detector and a fluoroscopy radiation generation device as one set and configured to capture a digital radiography image including the tracking target; and
a calculation device configured to obtain the position of the tracking target from the digital radiography image obtained by the digital radiography device, wherein
the calculation device
evaluates priority of visibility of the tracking target among a plurality of the digital radiography images of the tracking target,
calculates a weight distribution representing relative presence accuracy of the tracking target in a low-visibility digital radiography image using epipolar geometry and a difference in the visibility among the digital radiography images, and
determines a detection position of the tracking target in the low-visibility digital radiography image using the weight distribution and a similarity distribution of the low-visibility digital radiography image.

2. The tracking target position detection device according to claim 1, wherein
the calculation device reduces the weight distribution in a region outside a high accuracy region surrounded by epipolar lines based on an upper position of a similarity distribution of a high-visibility digital radiography image.

3. The tracking target position detection device according to claim 1, wherein
a calculation result of the detection position in the low-visibility digital radiography image is displayed on a display device using the weight distribution and the difference in the visibility among the plurality of digital radiography images.

4. The tracking target position detection device according to claim 1, wherein
the calculation device determines the visibility of the tracking target based on a similarity distribution of each of the digital radiography images.

5. A radiotherapy system comprising:
the tracking target position detection device according to any one of claims 1 to 4; and
an irradiation device configured to extract radiation for therapy.

6. A tracking target position detection method for detecting a position of a tracking target, the tracking target position detection method comprising:
evaluating priority of visibility of the tracking target among a plurality of digital radiography images of the tracking target;
calculating a weight distribution representing relative presence accuracy of the tracking target in a low-visibility digital radiography image using epipolar geometry and a difference in the visibility among the digital radiography images; and
determining a detection position of the tracking target in the low-visibility digital radiography image by using the weight distribution and a similarity distribution of the low-visibility digital radiography image.
